**Europäisches Patentamt**

**European Patent Office**

**Office européen des brevets**

(11) Veröffentlichungsnummer: **0 111 894**
**B1**

(12) **EUROPÄISCHE PATENTSCHRIFT**

(45) Veröffentlichungstag der Patentschrift:
04.09.85

(21) Anmeldenummer: 83112599.2

(22) Anmeldetag: 15.12.83

(51) Int. Cl.⁴: **C 07 C 21/12**, C 07 C 17/42

(54) Verfahren zum Stabilisieren von epoxidhaltigem Perchlorethylen.

(30) Priorität: 17.12.82 DE 3246886

(43) Veröffentlichungstag der Anmeldung:
27.06.84 Patentblatt 84/26

(45) Bekanntmachung des Hinweises auf die Patenterteilung:
04.09.85 Patentblatt 85/36

(84) Benannte Vertragsstaaten:
**BE DE FR GB IT NL**

(56) Entgegenhaltungen:
**DE - A - 2 853 848**

(73) Patentinhaber: **WACKER-CHEMIE GMBH,
Prinzregentenstrasse 22, D-8000 München 22 (DE)**

(72) Erfinder: **Blum, Klaus, Dr. Dipl.-Chem., Amatiweg 5,
D-8263 Burghausen (DE)**

ACTORUM AG

## Beschreibung

Die Erfindung betrifft ein Verfahren zum Stabilisieren von Perchlorethylen, das als Stabilisierungsmittel bekannte Epoxide enthält.

Insbesondere für die Zwecke der Metallentfettung wird hochstabilisiertes Perchlorethylen verwendet, das neben Antioxidantien auch Säurebindemittel aus der Gruppe der Epoxide enthält. Diese Epoxide sind jedoch ihrerseits anfällig für Umlagerungsreaktionen, die von den stets in im Gebrauch befindlichen Perchlorethylen in Spuren vorhandenen Lewissäuren, wie Zinkchlorid, Eisenchlorid, Aluminiumchlorid u.a. katalysiert werden. Bei diesen Umlagerungsreaktionen entstehen Produkte, die keine stabilisierende Wirkung besitzen. Der damit verbundene beschleunigte Abbau an Epoxid destabilisiert letztlich das in Gebrauch befindliche Perchlorethylen.

Ein typisches Beispiel für ein Epoxid, das zur Stabilisierung von Perchlorethylen eingesetzt wird, ist Cyclohexenoxid. Cyclohexenoxid lagert sich in den keine stabilisierende Wirkung aufweisenden Cyclopentylaldehyd um. Es ist bereits gemäss DE-OS 28 53 848 bekanntgeworden, die Umlagerungsreaktion mit 2.3-Epoxypropanol zu unterdrücken. Nachteiligerweise neigt 2.3-Epoxypropanol zu Polykondensationsreaktionen, wobei Produkte entstehen, die sich als schmierige Beläge am Reinigungsgut und an den Wänden der Reinigungskammern absetzen.

Aufgabe der Erfindung war es, epoxidhaltiges Perchlorethylen zu stabilisieren. Insbesondere war es Aufgabe der Erfindung, die unter dem Einfluss von Lewissäuren stattfindenden Umlagerungsreaktionen der Epoxide mindestens zurückzudrängen bzw. zu verhindern.

Gelöst wird diese Aufgabe durch Zusatz von 1.2-Epoxicyclooctan und/oder 1.2.5.6-Diepoxicyclooctan zum mit Epoxiden stabilisierten Perchlorethylen.

Gegenstand der Erfindung ist somit ein Verfahren zum Stabilisieren von Perchlorethylen, das als Stabilisierungsmittel bekannte Epoxide enthält, das dadurch gekennzeichnet ist, dass eine wirksame Menge an 1.2-Epoxicyclooctan und/oder 1.2.5.6-Diepoxicyclooctan zugesetzt wird.

Vorzugsweise werden 1.2-Epoxidcyclooctan und/oder 1.2-5.6-Diepoxicyclooctan in Mengen von 0,5 bis 10 Gew.%, besonders bevorzugt in Mengen von 1 bis 4 Gew.%, bezogen auf die Menge der sonst anwesenden Epoxide zugesetzt.

1.2-Epoxicyclooctan und 1.2-5.6-Diepoxicyclooctan sind an sich bekannte, handelsübliche Substanzen. Sie sind z.B. durch übliche Epoxidierungsreaktionen von Cycloocten bzw. Cyclooctadien zugänglich.

In der Praxis wird die Auswahl an als Säurebindemittel bekannten Epoxiden nicht zuletzt unter dem Gesichtspunkt getroffen, dass sich deren Siedepunkte um nicht mehr als 15° C vom Siedepunkt des stabilisierten Perchlorethylens unterscheiden. Typische Beispiele für derartige Epoxide sind Epoxibutan, 1.2-Epoxihexan und dessen Isomere, 1.2-Epoxioctan und dessen Isomere, Diisobutylenoxid, Cyclohexenoxid und 5.6-Epoxihexen-1.

Die genannten Epoxide werden in der Regel in Mengen von 1000 bis 5000 Gew.ppm., insbesondere in Mengen von 2000 bis 3000 Gew.ppm., jeweils bezogen auf das Gesamtgewicht des stabilisierten Perchlorethylens, eingesetzt.

Entsprechend ergibt sich, bezogen auf das Gesamtgewicht des erfindungsgemäss stabilisierten Perchlorethylens nach der bevorzugten Ausführungsform des Verfahrens eine Menge von 50 bis 500 Gew.ppm., insbesondere 80 bis 150 Gew.ppm., an 1.2-Epoxicyclooctan und/oder 1.2-5.6-Diepoxicyclooctan. Der Zusatz an erfindungsgemäss einzusetzenden Epoxiden erfolgt in der gleichen Weise, wie auch bereits bisher Epoxide dem zu stabilisierenden Perchlorethylen zugesetzt wurden.

Das erfindungsgemäss stabilisierte Perchlorethylen kann daneben auch jene stabilisierenden Zusätze enthalten, die auch bisher zur Stabilisierung von Perchlorethylen eingesetzt werden konnten. Es sind dies insbesondere

Amine, wie Triethylamin, Di-iso-propylamin, Dimethylisobutylamin, sec.-Butylamin, Pentylamin, Iso-pentylamin, 5-Methyl-2-hexan-amin, Di-iso-butylamin, N-Methylpyrrol, N-Methylmorpholin, u.a.

Ether, insbesondere Dialkylether, wie Dibutylether und Di-sec.-butylether, Dialkoxymethane, wie Dimethoxymethan und Diethoxymethan, Glykoldialkylether, wie Dimethoxyethan, Diethoxyethan und Butyl-glykol-tert.-butylether, Polyglykolether, wie Ethyl, Diglykol-tert.-butylether, Methoxy-diglykol-tert.-butylether, Triglykoldimethylether, Arylether, wie Diphenylether, Aralkylether, wie Dibenzylether, Arylalkylther, wie Anisol, Hydrochinondimethylether;

Olefine, wie Di-iso-butylen und Cycloheptatrien;

Alkylphenole, wie p-Kresol, o-Kresol, 2.6-Dimethylphenol, 2.4.6-Trimethylphenol, p-Isopropylphenol, p-tert.-Butylphenol, 2.6-Di-tert.-butylphenol, p-Amylphenol.

In dem erfindungsgemäss hergestellten Perchlorethylen liegt eine Epoxidkombination vor, bei der destabilisierende Umlagerungsreaktionen von Epoxiden auch in Gegenwart von Lewissäuren weitgehend unterdrückt werden. Es gelingt dadurch überraschenderweise durch Zusatz von lediglich geringen Mengen an weiterem Epoxid, das Säurebindevermögen von in Gebrauch befindlichem Perchlorethylen über eine grössere Zeitspanne zu erhalten, Korrosionsschäden zu vermeiden und die Bildung von Ablagerungen in den Vorratgefässen sowie am Reinigungsgut zu verhindern.

Die Erfindung wird nun anhand von Beispielen und Vergleichsbeispielen näher erläutert:

*Beispiel 1*

Perchlorethylen mit einem Gehalt an
   100 Gew.ppm 1.2.5.6-Diepoxicyclooctan
   2500 Gew.ppm 1.2-Epoxicyclohexan
   15 Gew.ppm Di-iso-propylamin

25 Gew.ppm 2.4-Di-tert.-Butylphenol
50 Gew.ppm N-Methylmorpholin
wurde mit 200 Gew.ppm wasserfreiem Zinkchlorid versetzt und 72 Stunden am Rückfluss gekocht. Das Säurebindevermögen wurde durch die Salzsäureadditionsfähigkeit nach der folgenden Methode gemessen: als Hydrochlorierungsreagens diente eine Lösung von Salzsäure in Isopropanol (4,4 cm³ konzentrierte Salzsäure zu 500 cm³ ergänzt mit Isopropanol). Der Titer dieser Lösung wurde mit 0,1 N NaOH gegen Bromphenolblau ermittelt (Verbrauch A).

Es wurden nun 25 cm³ Hydrochlorierungsreagens, 10 cm³ des oben beschriebenen Perchlorethylens und 25 cm³ Isopropanol geschüttelt und 10 Minuten bei Raumtemperatur stehengelassen. Danach wurde mit 0,1N NaOH gegen Bromphenolblau zurücktitriert (Verbrauch B).

Das Säurebindevermögen wurde nach der folgenden Formel berechnet:

$$\frac{(A-B)\cdot 4,0\cdot 100\cdot F}{V\cdot D\cdot 1000} = \% \ NaOH$$

4,0 = Wirkungswert der 0,1 N NaOH in mg/cm³
F = Faktor der 0,1 NaOH
V = Volumen des eingesetzten Perchlorethylens in cm³
D = Dichte des Perchlorethylens in g/cm³
Ergebnis:
Das Säurebindevermögen betrug 0,093 Gew.% NaOH

*Vergleichsbeispiel 1*

Es wurde die Arbeitsweise gemäss Beispiel 1 wiederholt, mit der Abänderung, dass ein ansonsten gleichermassen stabilisiertes Perchlorethylen ohne Zusatz von 1.2-5.6-Diepoxycyclooctan verwendet wurde.
Ergebnis:
Das Säurebindevermögen betrug 0 Gew.% NaOH

*Beispiel 2*

Die Arbeitsweise gemäss Beispiel 1 wurde wiederholt, mit der Abänderung, dass anstatt des Zusatzes von 2500 Gew.ppm 1.2-Epoxicyclohexan 2500 Gew.ppm eines 4:1-Gemisches von 2.3-Epoxihexan und 3.4-Epoxihexan verwendet wurde.
Das entsprechend stabilisierte Perchlorethylen wies ein Säurebindevermögen von 0,083 Gew.% NaOH auf.

*Vergleichsbeispiel 2*

Die Arbeitsweise gemäss Beispiel 2 wurde wiederholt, mit der Abänderung, dass der Zusatz an 100 Gew.-ppm 1.2-5.6-Diepoxycyclooctan unterblieb.
Das Säurebindevermögen betrug 0 Gew.% NaOH

*Beispiel 3*

Die Arbeitsweise gemäss Beispiel 1 wurde wiederholt, mit der Abänderung, dass anstatt 1.2-Epoxicyclohexan 2500 Gew.ppm 1.2-Epoxihexan verwendet wurden.
Das Säurebindevermögen betrug 0,078 Gew.% NaOH

*Vergleichsbeispiel 3*

Die Arbeitsweise gemäss Beispiel 3 wurde wiederholt, mit der Abänderung, dass der Zusatz von 100 Gew.ppm 1.2.5.6-Diepoxicyclooctan unterblieb.
Das Säurebindevermögen betrug 0 Gew.% NaOH

*Beispiel 4*

Die Arbeitsweise gemäss Beispiel 1 wurde wiederholt, mit der Abänderung, dass anstatt des Zusatzes von 100 Gew.ppm 1.2-5.6-Diepoxicyclooctan 100 Gew.-ppm 1.2-Epoxicyclooctan verwendet wurden.
Ergebnis:
Das Säurebindevermögen betrug 0,078 Gew.% NaOH

*Beispiel 5*

Proben stabilisierten Perchlorethylens wurden in einen 400 ml Dreihalskolben mit Rückflusskühler gebracht und Metallstreifen aus Kupfer, Messing, Zink, Aluminium und Stahl innerhalb der Flüssigkeit so angeordnet, dass die Metallstreifen zur Hälfte in die Flüssigkeit eintauchten. Es wurde 168 Stunden am Rückfluss gekocht.

Danach wurden die Metallstreifen auf Korrosion und Belagbildung bonitiert. Ferner wurden Proben des wie oben beschrieben behandelten Perchlorethylens mit gleichen Volumteilen Wasser versetzt, intensiv geschüttelt und danach der pH-Wert der wässerigen Phase bestimmt.
Ergebnisse:
Beim gemäss Beispiel 1 beschriebenen Perchlorethylen wurde ein pH-Wert von 7,7 ermittelt. Die Metallstreifen waren nicht korrodiert.

Beim gemäss Beispiel 2 beschriebenen Perchlorethylen betrug der pH-Wert 8,3. Keine Korrosion an den Metallstreifen.

Beim gemäss Beispiel 3 beschriebenen Perchlorethylen betrug der pH-Wert 8,3. Keine Korrosion an den Metallstreifen.

*Vergleichsbeispiel 4*

Es wurde die Arbeitsweise gemäss Beispiel 5 Proben Perchlorethylens wiederholt, die gemäss den Vergleichsbeispielen 1, 2 und 3 beschrieben sind.
Bei einer Probe Perchlorethylens gemäss Vergleichsbeispiel 1 wiesen alle Metallstreifen einen klebrigen Belag auf und waren ankorrodiert. Der pH betrug 4,2.

Bei der Probe gemäss Vergleichsbeispiel 2 waren sämtliche Metallstreifen (sowohl die Teile, die in die Flüssigkeit eintauchten als auch die Teile, die über die Flüssigkeit hinausragten) ankorrodiert. Der pH-Wert betrug 4,3.

Bei der Probe gemäss Vergleichsbeispiel 3 waren sämtliche Metallstreifen ankorrodiert. Lediglich die Metallstreifen aus Zink und Aluminium blieben in dem Teil blank, der über die Flüssigkeit hinausragte. Der pH-Wert betrug 5,1.

## Patentansprüche

1. Verfahren zum Stabilisieren von Perchlorethylen, das als Stabilisierungsmittel bekannte Epoxide enthält, dadurch gekennzeichnet, dass eine wirksame Menge an 1.2-Epoxicyclooctan und/oder 1.2-5.6-Di-epoxicyclooctan zugesetzt wird.

2. Verfahren nach Anspruch 1, dadurch gekennzeichnet, dass 1.2-Epoxicyclooctan und/oder 1.2-5.6-Diepoxicyclooctan in Mengen von 0,5 bis 10 Gew.-%, bezogen auf die Menge der sonst anwesenden Epoxide zugesetzt werden.

## Claims

1. Process for stabilising perchloroethylene which contains known epoxides as stabilising agents, characterised in that an effective amount of 1,2-epoxycyclooctane and/or 1,2-5,6-di-epoxycyclooctane is added.

2. Process according to claim 1, characterised in that 1,2-epoxycyclooctane and/or 1,2-5,6-diepoxycyclooctane is added in amounts of from 0.5 to 10% by weight, based on the amount of the other epoxides present.

## Revendications

1. Procédé de stabilisation de perchloréthylène contenant comme stabilisants des époxydes connus, procédé caractérisé en ce que l'on ajoute au perchloréthylène une proportion appropriée de 1,2-époxycyclooctane et/ou de 1,2-5,6-diépoxy-cyclooctane.

2. Procédé selon la revendication 1, caractérisé en ce que l'on ajoute au perchloréthylène 0,5 à 10% en poids de 1,2-époxycyclooctane et/ou de 1,2-5,6-diépoxycyclooctane, par rapport à la quantité des autres époxydes présents.